# EUROPÄISCHE PATENTSCHRIFT

(11) **EP 0 465 408 B1**
(45) Veröffentlichungstag und Bekanntmachung des Hinweises auf die Patenterteilung: **14.09.1994**
(21) Anmeldenummer: 91810431.6
(22) Anmeldetag: 07.06.1991
(51) Int. Cl.: A61F 2/08

(54) **Bandverankerung**
Ligament anchor
Ancrage pour ligament

(30) Priorität: 06.07.1990 CH 2242/90
(43) Veröffentlichungstag der Anmeldung: 08.01.1992
(73) Patentinhaber: SULZER Medizinaltechnik AG, 8404 Winterthur (CH); PROTEK AG, 3110 Münsingen-Bern (CH)
(72) Erfinder: Mansat, Christian, Dr.-med., F-31130 Balma (FR); Frey, Otto, Dr., CH-8400 Winterthur (FR); Roland, Willi, CH-8543 Stadel (CH)
(74) Vertreter: Trieblnig, Adolf

(56) Entgegenhaltungen:
- EP-A- 0 232 049
- EP-A- 0 342 281
- DE-U- 8 914 308
- FR-A- 2 395 012
- FR-A- 2 586 927

## Beschreibung

Die Erfindung betrifft eine Bandverankerung zur Befestigung von implantiertem Bandersatz im Knochengewebe nach dem Oberbegriff des Anspruchs 1. Eine solche Bandverankerung ist aus der DE-U-8914308 bekannt.

Biegsamer Sehnen- oder Bandersatz ist auch aus den Patentschriften CH 644 748 und EP-A-0 067 929 bekannt. Bisher wurden künstliche Bänder nach dem Einziehen durch eine Oeffnung im Knochengewebe auf der äusseren Knochenoberfläche mit Heftklammern fixiert. Durch das Heften entstehen oft Verletzungen oder überhöhte Spannungsspitzen am eingesetzten Band, die seine langfristige Funktion einschränken. Die Patentanmeldung FR 78 18385 zeigt eine aussen konische Verankerungshülse, die in der Oeffnung des Knochengewebes eingesetzt ist und die einen Querstift trägt, über welchen das künstliche Band in Form einer festen Schlaufe gezogen ist, um den Bandzug zu übertragen. Das Erreichen einer passenden Bandlänge erfordert hier umfangreiche Vorarbeiten und grosses Geschick.

Hier schafft die Erfindung Abhilfe. Sie löst die Aufgabe, ein im Knochen eingezogenes Band unter einem definierten Bandzug schonend zu fixieren. Gemäss der Erfindung wird die Aufgabe durch die in dem Anspruch 1 enthaltenen Merkmale gelost.

Die Vorteile der Erfindung sind darin zu sehen, dass beim Setzen und eventuellen Nachspannen des künstlichen Bandes keinerlei Risiken eingegangen werden, die ein Auswechseln des Bandes wegen Verletzung oder wegen nicht zutreffender Länge notwendig machen. Die abhängigen Ansprüche 2 bis 6 beziehen sich auf vorteilhafte Weiterbildungen der Erfindung.

Im folgenden wird die Erfindung anhand von einem Ausführungsbeispiel beschrieben. Es zeigen:
- Fig. 1: Entgegen der Belastungsrichtung die Draufsicht auf eine aussen konische Verankerungshülse mit Längsrippen;
- Fig. 2: die geschnittene Seitenansicht einer angeschrägten und im Knochengewebe eingesetzten Verankerungshülse gemäss Fig. 1;
- Fig. 3: entgegen der Belastungsrichtung die Draufsicht auf eine Klemmhülse, die radial auf einen kleineren Innendurchmesser deformierbar ist;
- Fig. 4: in Belastungsrichtung die Draufsicht auf eine Klemmhülse gemäss Fig. 3;
- Fig. 5: die geschnittene Seitenansicht einer Klemmhülse gemäss Fig. 4;
- Fig. 6: die Seitenansicht einer Klemmhülse gemäss Fig. 3;
- Fig. 7: einen vergrösserten Schnitt der Sägezahnstruktur auf der Innenfläche einer Klemmhülse.

Die Figuren zeigen eine Bandverankerung zur Befestigung von implantiertem Bandersatz im Knochengewebe 28, wobei ein Band durch eine Oeffnung im Knochengewebe geführt ist. Die Bandverankerung besteht einerseits aus einer in Belastungsrichtung 15 aussen konisch zulaufenden Klemmhülse 20, die radial auf einen kleineren Innendurchmesser deformierbar ist und die auf ihrer Innenwand sägezahnähnlich vorstehende Segmente 25 mit steilen Flanken 24 entgegen der Belatungsrichtung 15 besitzt, welche in das eingezogene Band eingreifen. Andererseits wird die Klemmhülse 20 durch eine in Belastungsrichtung 15 innen konisch zulaufende Veranerkungshülse 1 abgestützt, welche in die Oeffnung im Knochengewebe 28 eingesetzt ist. Zur Verankerung wird das Band unter Vorspannung entgegen der Belastungsrichtung 15 nach aussen gezogen und gleichzeitig wird die Klemmhülse 20 in Belastungsrichtung 15 soweit in die Verankerungshülse 1 eingepresst, dass die Sägezahnelemente 25 das Band so fest umschliessen und halten, dass unter dem aufgebauten Druck und dem Bandzug in Belastungsrichtung 15 eine Selbsthemmung der konischen Verbindung 4, 17 stattfindet.

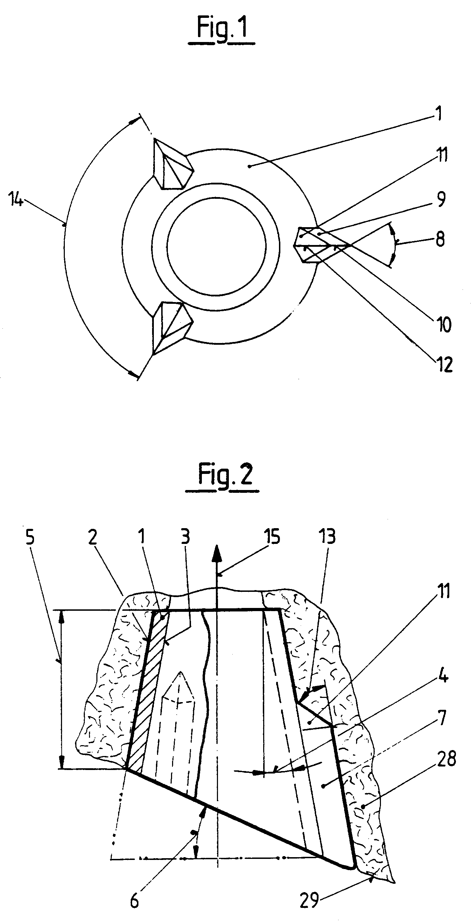

In den Fig. 1 und 2 ist eine im Knochengewebe 28 eingesetzte Verankerungshülse 1 zu sehen, deren Anschrägung 6 mit der Knochenoberfläche 29 abschliesst. Die Verankerungshülse 1 besitzt einen Aussenkonus 2, der sich in Belastungsrichtung 15 auf einer entsprechenden konischen Bohrung im Knochengewebe 28 abstützt. Auf dem Aussenkonus 2 sind Längsrippen 7 mit Schneidkante 10 und Seitenflächen 9 im Schnittwinkel 8 angebracht, die in Belastungsrichtung 15 in einer Querschneide 12 mit Seitenflächen 11 und einem Knickwinkel 13 enden, um sich zur Drehsicherung und Primärverankerung im Knochengewebe 28 einzugraben. Die gezeigten drei Längsrippen stehen in einem Spreizwinkel 14 von 120° zueinander. Sie können durch wesentlich mehr, enger stehende und weniger stark vorstehende Rippen ersetzt werden. Die durch die Anschrägung 6 reduzierte Hülsenlänge 5 ist grösser als die Länge 16 der Klemmhülse 20, damit diese vollständig eintaucht.

Auf ihrer Innenseite besitzt die Verankerungshülse 1 einen Innenkonus 3, dessen halber Konuswinkel 4 mit dem halben Konuswinkel 17 am Aussenkonus der Klemmhülse 20 übereinstimmt. Die Konusflächen sind mit einem halben Konuswinkel 4, 17 in der Grössenordnung von 10° versehen und sie sind soweit aufgerauht, dass beim Ineinanderschieben unter Vorspannung Selbsthemmung entsteht.

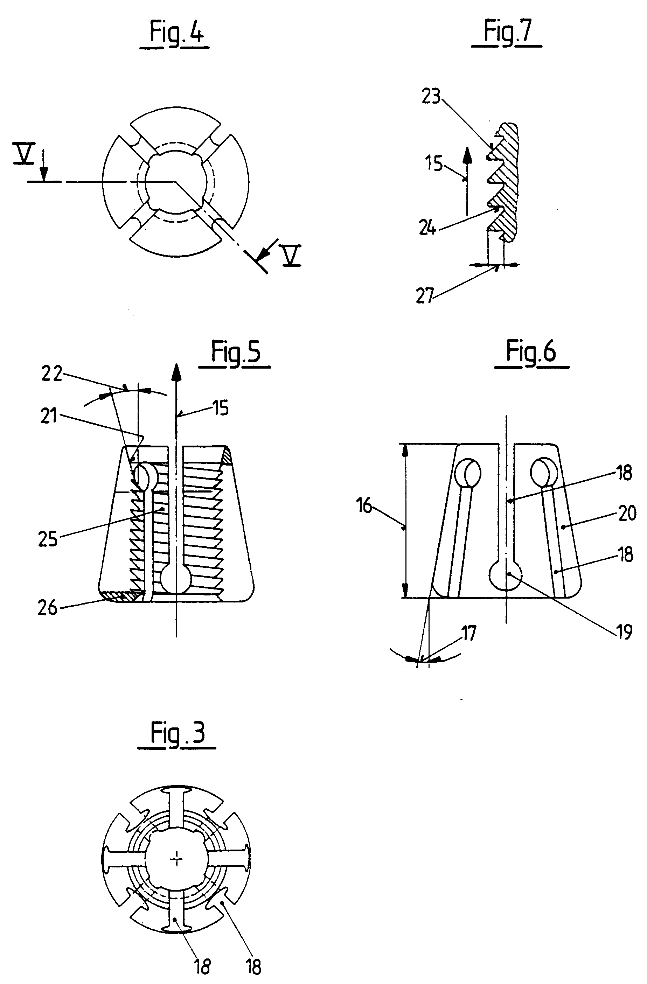

In den Fig. 3 bis 7 ist eine Klemmhülse 20 gezeigt, deren Mantelfläche von Schlitzen 18 unterbrochen ist, welche wechselseitig an den Stirnflächen der Klemmhülse beginnen und zur entgegengesetzten Stirnfläche nur einen kleinen Restquerschnitt 26 am Schlitzgrund stehen lassen. Der Schlitzgrund weisst eine Rundung 19 auf, die eine Verengung des Schlitzes zur Verringerung des Innendurchmessers der Klemmhülse 20 zulässt.

Zum Einziehen eines künstlichen Bandes entgegen der Belastungsrichtung 15 ist die Klemmhülse 20 mit einer konischen Einlaufstrecke 21 entsprechend einem halben Konuswinkel 22 versehen, welcher die Durchmesserunterschiede zwischen ungespanntem und gespanntem Band berücksichtigt. Die anschliessenden sägezahnähnlich nach innen vorstehenden Segmente 25 sind Ausschnitte eines Innengewindes mit einer Sägezahnhöhe 27, die auf eine am Band zulässige Eindringtiefe abgestimmt ist. Beim Bandeinzug entgegen der späteren Belastungsrichtung 15 rutscht das Band über flache Nachrutschflanken 23 und verrundete Gewindespitzen. Zum Setzen der Klemmhülse 20 wird zunächst das Band z.B. mit einer Federwaage auf eine vorgesehene Spannung gebracht und anschliessend die Klemmhülse 20 mit einem Setzinstrument gegen die Verankerungshülse 1 gepresst, damit sich beim Verkleinern ihres Innendurchmessers die sägezahnähnlichen Segmente 25 in das Band einpressen und eine Vorspannung aufbauen können, die zur Selbsthemmung der Konen 4, 17 führt. Die Selbsthemmung bleibt nach dem Entfernen des Setzwerkzeuges erhalten und wird mit der späteren Bandbelastung langzeitig immer wieder sichergestellt. Eine Wiederholung des Setzvorganges oder ein späteres Nachspannen sind möglich, solange das verankerte Band soweit aus der Klemmhülse 20 heraussteht, dass es greifbar ist, um die Selbsthemmung der Konen 4, 17 zu überwinden und um eine vorgeschriebene Vorspannung vor dem neuen Einpressen der Klemmhülse 20 aufzubringen.

## Patentansprüche

1. Bandverankerung zur Befestigung von implantiertem Bandersatz im Knochengewebe, wobei ein Band durch eine Öffnung im Knochengewebe (28) geführt ist, die an der äusseren Knochenoberfläche (29) endet, wobei die Bandverankerung aus einer in einer Belastungsrichtung (15) aussen konisch zulaufenden Klemmvorrichtung (20) besteht, die radial auf einen kleineren Durchmesser verstellbar ist und die auf ihrer Innenwand sägezahnähnlich vorstehende Segmente (25) aufweist, welche steile Flanken (24) entgegen der Belastungsrichtung (15) besitzen, wobei zur selbsthemmenden Verankerung der Klemmvorrichtung (20) eine in Belastungsrichtung (15) innen konisch zulaufende Verankerungshülse (1) in die Öffnung im Knochengewebe (28) eingesetzt ist, dadurch gekennzeichnet, dass die Klemmvorrichtung eine einteilige Klemmhülse (20) ist, die radial auf einen kleineren Durchmesser deformierbar ist.

2. Bandverankerung nach Anspruch 1, dadurch gekennzeichnet, dass die Sägezahnsegmente (25) Ausschnitte eines Innengewindes mit Sägezahnform sind.

3. Bandverankerung nach Anspruch 1, dadurch gekennzeichnet, dass der Aussenkonus der Klemmhülse (20) und der Innenkonus (3) der Verankerungshülse (1) einen halben Konuswinkel (4, 17) in der Grössenordnung von 10 ° aufweisen und dass die Konusflächen soweit aufgerauht sind, dass Selbsthemmung beim Ineinanderschieben eintritt.

4. Bandverankerung nach Anspruch 1, dadurch gekennzeichnet, dass die Klemmhülse (20) auf ihrer Innenseite entgegen der Belastungsrichtung (15) einen sich verjüngenden Bandeinlauf (21) aufweist.

5. Bandverankerung nach Anspruch 1, dadurch gekennzeichnet, dass eine aussen konische Verankerungshülse (1) mit Längsrippen (7) versehen ist, die sich zur Verdrehsicherung in das Knochengewebe (28) eingraben.

6. Bandverankerung nach Anspruch 1, dadurch gekennzeichnet, dass die Verankerungshülse (1) eine Anschrägung (6) aufweist, die der äusseren Form einer Knochenoberfläche (29) angepasst ist, und dass ihre reduzierte Hülsenlänge (5) mindestens der Länge der Klemmhülse (20) entspricht.

## Claims

1. A ligament anchor for fastening implanted artificial ligament into bone tissue, where a ligament is led through an opening in the bone tissue (28) which ends at the outer surface (29) of the bone, the ligament anchor consisting of a clamping device (20) which on the outside tapers in conically in a direction (15) of loading and is adjustable radially to a smaller diameter and exhibits on its inner wall projecting sawtooth segments (25) which have steep flanks (24) against the direction (15) of loading, and an anchoring sleeve (1) which on the inside tapers in conically in the direction (15) of loading being inserted into the opening in the bone tissue (28) for the self-locking anchoring of the clamping device (20), characterized in that the clamping device is a one-piece clamp sleeve (20) which is radially deformable to a smaller diameter.

2. A ligament anchor as in Claim 1, characterized in that the sawtooth segments (25) are segments of an internal thread of sawtooth shape.

3. A ligament anchor as in Claim 1, characterized in that the outer cone of the clamp sleeve (20) and the inner cone (3) of the anchoring sleeve (1) exhibit a half-apex-angle (4, 17) of the order of magnitude of 10° and that the faces of the cones are roughened enough for self-locking to occur upon sliding them together.

4. A ligament anchor as in Claim 1, characterized in that the clamp sleeve (20) exhibits on the inside a ligament entry (21) which tapers in against the direction (15) of loading.

5. A ligament anchor as in Claim 1, characterized in that an anchoring sleeve (1) which is conical on the outside is provided with longitudinal ribs (7) which dig into the bone tissue (28) for security against twisting.

6. A ligament anchor as in Claim 1, characterized in that the anchoring sleeve (1) exhibits a bevel (6) which is adapted to the outer shape of a surface (29) of the bone, and that its reduced sleeve length (5) corresponds with at least the length of the clamp sleeve (20).

## Revendications

1. Ancrage de ligament pour la fixation d'une prothèse implantée de ligament dans le tissu osseux, un ligament étant introduit dans un trou du tissu osseux (28) qui aboutit à la surface extérieure (29) de l'os, l'ancrage de ligament se composant d'un dispositif de serrage (20) qui se rétrécit en cône extérieurement dans un sens (15) de charge, qui est ajustable radialement sur un plus petit diamètre et qui comporte sur sa paroi intérieure des segments (25) en saillie qui sont analogues à des dents de scie et qui ont des flancs (24) en pente raide dans le sens opposé à celui de la charge (15), un manchon d'ancrage (1) qui se rétrécit en cône intérieurement dans le sens (15) de charge étant installé dans le trou du tissu osseux (28) pour l'ancrage à autoblocage du dispositif de serrage (20), caractérisé en ce que le dispositif de serrage est un manchon monobloc de serrage (20) qui est déformable radialement dans le sens de réduction de son diamètre.

2. Ancrage de ligament selon la revendication 1, caractérisé en ce que les segments (25) en dents de scie sont des parties d'un taraudage à forme en dents de scie.

3. Ancrage de ligament selon la revendication 1, caractérisé en ce que le cône extérieur du manchon de serrage (20) et le cône intérieur (3) du manchon d'ancrage (1) ont un demi-angle de cône (4, 17) qui est d'un ordre de grandeur de 10° et en ce que les surfaces coniques sont rendues suffisamment rugueuses pour que se produise un autoblocage lorsqu'ils sont enfilés l'un dans l'autre.

4. Ancrage de ligament selon la revendication 1, caractérisé en ce que le manchon de serrage (20) comporte du côté intérieur une entrée (21) de ligament qui se rétrécit dans le sens inverse à celui de la charge (15).

5. Ancrage de ligament selon la revendication 1, caractérisé en ce qu'un manchon d'ancrage (1) extérieurement conique comporte des nervures longitudinales (7) qui s'incrustent dans le tissu osseux (28) pour interdire toute rotation.

6. Ancrage de ligament selon la revendication 1, caractérisé en ce que le manchon d'ancrage (1) comporte un biais (6) qui est adapté à la forme extérieure de la surface (29) de l'os et en ce que la longueur réduite (5) de ce manchon correspond au moins à la longueur du manchon de serrage (20).
